# EUROPÄISCHE PATENTSCHRIFT

(11) **EP 1 597 999 B1**
(45) Veröffentlichungstag und Bekanntmachung des Hinweises auf die Patenterteilung: **20.08.2008**
(21) Anmeldenummer: 04011644.4
(22) Anmeldetag: 17.05.2004
(51) Int. Cl.: A61B 1/00

(54) **Endoskopisches Instrument**
Endoscopic device
Dispositif endoscopique

(43) Veröffentlichungstag der Anmeldung: 23.11.2005
(73) Patentinhaber: Richard Wolf GmbH, 75438 Knittlingen (DE)
(72) Erfinder: Bonnet, Ludwig, 75438 Knittlingen (DE); Hipp, Klaus-Peter, 75038 Grossvillars (DE); Ernst, Thomas, 75057 Kürnbach (DE); Fritz, Michael, 76199 Karlsruhe (DE)
(74) Vertreter: Vollmann, Heiko

(56) Entgegenhaltungen:
- US-A- 3 774 596
- US-A1- 2001 056 224

## Beschreibung

Die Erfindung betrifft ein endoskopisches Instrument mit den im Oberbegriff des Anspruchs 1 angegebenen Merkmalen.

Eine zunehmende Anzahl diagnostischer und therapeutischer Eingriffe wird heutzutage mittels minimal-invasiver Techniken durchgeführt. Die dabei eingesetzten endoskopischen Instrumente werden durch Körperöffnungen, bei denen es sich um natürliche Körperöffnungen, aber auch um künstliche, durch Punktion geschaffene Kanäle handeln kann, zum Einsatzort in das Körperinnere geschoben. Während der Zeit des Eingriffs liegt die Außenwand des Endoskopschafts teilweise eng an dem Körpergewebe des Öffnungskanals an, so dass bei länger dauernden Eingriffen die Gefahr besteht, dass das Körpergewebe bzw. die Schleimhaut an dem Schaft anhaftet und bei anwendungsbedingten Bewegungen des Endoskopschafts von diesem abreißt und traumatisiert wird.

Aus diesem Grund ist es üblich, die Kontaktfläche zwischen Schaft und umgebenden Körpergewebe mit einer Gleitmittelschicht zu versehen, um so das Anhaften des Körpergewebes an dem Schaft zu verhindern. Dabei kann das Gleitmittel vor dem Eingriff auf den Schaft des endoskopischen Instruments aufgetragen werden, oder in die betreffende Körperöffnung eingespritzt werden.

Aus DE 101 11 354 A1 ist ein endoskopisches Instrument bekannt, das Versorgungskandle aufweist, über die die Außenfläche des Schafts mit einem fließfähigen Gleitmittel versorgt wird. Die Gleitmittelzufuhr erfolgt während des Eingriffs kontinuierlich über einen Versorgungsanschluss, der am proximalen Ende des Schafts angeordnet ist. Die Versorgungskanäle sind im Inneren des Schafts angeordnet oder nutförmig in die Außenwand des Schafts eingearbeitet, wobei der Schaft einen schlauchförmigen Überzug aufweist, der die Versorgungskanäle gewebeseitig abschließt und eine Kontaktfläche zum Körpergewebe ausbildet. Die Kontaktfläche des Schafts zum umgebenden Gewebe ist mittels Durchbrechungen mit den Versorgungskanälen verbunden und wird so mit dem Gleitmittel versorgt. Nachteilig bei diesem endoskopischen Instrument ist es, dass die Reibungsverringerung ausschließlich durch den Einsatz eines Gleitmittels erfolgt, das der Schaftoberfläche während des Eingriffs zugeführt werden muss. Die Versorgungskanäle und der Versorgungsanschluss benötigen Raum und vergrößern den Außendurchmesser des Schafts, was die Gefahr einer Traumatisierung des umgebenden Gewebes erhöht. Des Weiteren ist ein erheblicher Herstellungsaufwand zum Ausbilden der Gleitmittelversorgung bei diesem endoskopischen Instrument erforderlich.

Bei Spekula, die in natürliche Körperhöhlen eingeführt werden, ist es üblich, diese so auszubilden, dass sie die Körperhöhlen beim Einführen gar nicht oder nur in geringem Maße aufweiten. So beschreibt US 3,774,596 ein Spekulum, welches zusammengefaltet in eine Körperhöhle eingebracht werden kann und dort mittels eines Druckgassystems entfaltet werden kann und anschließend den Öffnungsbereich einer Körperöffnung aufweitet. Wegen der geringen Schaftquerschnitte ist eine solche Ausgestaltung bei endoskopischen Schaftinstrumenten in der Regel nicht möglich.

Vor diesem Hintergrund liegt der Erfindung die Aufgabe zugunde, ein endoskopisches Instrument so zu modifizieren, dass Eingriffe mit diesem Instrument für den Patienten schonender gestaltet werden können und das endoskopische Instrument herstellungstechnisch einfach zu realisieren ist.

Diese Aufgabe wird erfindungsgemäß durch ein endoskopisches Instrument mit den im Anspruch 1 angegebenen Merkmalen gelöst. Vorteilhafte Weiterbildungen der Erfindung ergeben sich aus den Unteransprüchen, der nachfolgenden Beschreibung und der Zeichnung.

Das erfindungsgemäße endoskopische Instrument besitzt einen zum Einführen in eine Körperöffnung vorgesehen Schaft. Die Außenkontur des Schaftquerschnitts weist Erhebungen auf, die auf den Eckpunkten eines Polygons liegen, so dass ein im Wesentlichen linienförmiger Kontakt zwischen Schaft und Körper gebildet wird. Die polygone Querschnittsfläche des Schafts kann dabei eine beliebige Anzahl von Ecken aufweisen.

Die Gefahr einer Austrocknung und Anhaftung des Körpergewebes an einem Endoskopschaft ist umso größer, je größer die Kontaktfläche zwischen dem Schaft und dem umgebenden Körpergewebe ist. Bei dem erfindungsgemäßen endoskopischen Instrument kontaktieren nur die Scheitelbereiche der an dem Schaft angeordneten Erhebungen die Wandung des Körperkanals, während die übrige Schaftoberfläche in keinem Kontakt zu dem Körpergewebe steht. Die linienförmigen Kontaktflächen, die von den Erhebungen des Endoskopschafts mit dem umgebenden Körpergewebe ausgebildet werden, sind signifikant kleiner als die Kontaktflächen, die von bekannten zylindrischen Schäften gleicher radialer Querschnittsausdehnung ausgebildet werden. Demgemäß wird die Gefahr der Anhaftung bzw. der Austrocknung des Gewebes durch das erfindungsgemäße endoskopische Instrument deutlich verringert und der Schaft weist entsprechend bessere Gleiteigenschaften auf, so dass es in vielen Fällen, z.B. beim Führen des Schafts in einer Harnröhre, nicht erforderlich ist, die Schaftoberfläche mit einem Gleitmittel zu beaufschlagen, da der körpereigene Feuchtigkeitsfilm aufgrund des kleinen Kontaktbereichs allein in der Lage ist, ein Austrocknen des Gewebes zu verhindern.

Dennoch kann es vorteilhaft sein, auf den Schaft des endoskopischen Instruments vor dem Eingriff ein Gleitmittel aufzutragen. Auf diese Weise kann ein Austrocknen von Schleimhaut bzw. Körpergewebe und damit ein Anhaften während eines Eingriffs nahezu ausgeschlossen werden.

Erfindungsgemäß sind die Flächen zwischen den Erhebungen im Querschnitt zumindest teilweise konkav gekrümmt. Die zwischen den Erhebungen liegenden Oberflächenbereiche des Schafts sind so als abgerundete u-förmige Vertiefungen ausgebildet, die sich vorzugsweise in Längsrichtung des Schafts erstrecken und große Bereiche der Schaftoberfläche noch weiter von dem Körperkanal in Richtung der Schaftlängsachse beabstanden, was deren Kontakt mit dem Körperkanal nahezu ausschließt. Des Weiteren bilden die Vertiefungen ideale Depots für ein vor dem Eingriff aufzutragendes Gleitmittel.

Abhängig von der Anzahl der Ecken, die die Außenkontur der Querschnittsfläche des Schafts aufweist, sind die von den Ecken gebildeten Erhebungen an der Schaftoberfläche mehr oder weniger scharfkantig, wobei eine abnehmende Eckenzahl zu spitzer zulaufenden Erhebungen, d.h. scharfkantigeren Kontaktbereichen zwischen Schaft und Körpergewebe führt. Um dies zu vermeiden ist es zweckmäßig, die Erhebungen an der Oberfläche des Schafts abgerundet auszubilden. Auf diese Weise wird verhindert, dass Schaftbewegungen in einem Körperkanal eine Traumatisierung des diesen Kanal umgebenden Körpergewebes hervorrufen.

Bevorzugt weist der Schaft des endoskopischen Instruments an seinem Außenumfang ein Wellenprofil auf, wobei die Wellenberge im Wesentlichen parallel zur Längsachse des Schafts verlaufen.

Sind die Erhebungen des Schafts abgerundet ausgestaltet und die Flächen zwischen den Erhebungen konkav gekrümmt, können der Radius der Eckenabrundung und der Krümmungsradius der zwischen den Ecken liegenden Flächen so aufeinander abgestimmt werden, dass die Außenkontur des Schaftquerschnitts umfangsseitig als ein harmonisches Wellenprofil ausbildet, bei dem sinusförmig wechselnd Wellenberge und Wellentäler aufeinanderfolgen. Dabei weisen die durch die Wellenber ge gebildeten Erhebungen und die durch die Wellentäler gebildeten Vertiefungen über den gesamten Schaftumfang die gleiche Ausdehnung in Richtung der Schaftlängsachse auf. So variiert die radiale Querschnittsausdehnung des Schafts über dessen Umfang periodisch zwischen einem größten Wert an den Scheitelpunkten der Wellenberge und einem kleinsten Wert an den Scheitelpunkten der Wellentäler.

Da der Schaftquerschnitt vorzugsweise über die gesamte Schaftlänge in der oben beschriebenen Form ausgebildet ist, ergibt sich an der Schaftoberfläche eine periodisch wechselnde und sich in Richtung der Schaftlängsachse erstreckende Folge von Wellenbergen, die kammartige Erhebungen bilden und Wellentäler die in Richtung der Schaftlängsachse eingerückte Senken formen.

Auf diese Weise bilden die Scheitellinien der Wellenberge den Kontaktbereich zwischen dem Schaft und dem umgebenden Körpergewebe. Wird das endoskopische Instrument während eines Eingriffs in einem Körperkanal in Richtung der Schaftlängsachse bewegt, kommen nur schmale linienförmige Bereiche der Innenwand des Körperkanals mit der Oberfläche des Schafts in Berührung. Auch bei Drehbewegungen des Schafts in dem Körperkanal weist das erfindungsgemäße endoskopische Instrument deutlich bessere Gleiteigenschaften als bekannte Schaftausbildung auf. Ein Wellenprofil des Schaftes ist auch fertigungstechnisch besonders günstig, da ein solches Wellenprofil in einfacher Weise durch Verformung der Schaftwandung beispielsweise durch Ziehen, Drücken oder Prägen eines zylindrischen Rohres gebildet werden kann.

Die im Wesentlichen linienförmigen Kontaktbereiche des erfindungsgemäßen Schafts beschränken sich jedoch nicht auf das vorgenannte achsparallel verlaufende Wellenprofil, sondern können je nach Anforderung auch anders ausgebildet sein. So können die Erhebungen über den Schaftumfang beispielsweise auch verschränkt angeordnet sein, so dass sich eine schraubenlinienförmige Anordnung der linienförmigen Kontaktbereiche am Schaft ergibt. Eine solche Schaftausbildung ist insbesondere dann von Vorteil, wenn eine kombinierte Axial- und Drehbewegung, d. h. eine schraubenlinienförmige Bewegung des Schaftes innerhalb des Körperkanals vorgesehen ist.

Vorzugsweise gleichmäßig über den Umfang verteilt bilden an der Schaftoberfläche 5 bis 20 Erhebungen die linienförmigen Kontaktbereiche mit dem Körperkanal. Die Anzahl der Erhebungen ist abhängig von der Größe des Schaftquerschnitts, so dass Schäfte mit einer größeren radialen Querschnittsausdehnung an ihrem Außenumfang mehr Erhebungen aufweisen können als Schäfte mit einer kleineren radialen Querschnittsausdehnung. Die äußere radiale Schaftausdehnung liegt bei dem erfindungsgemäßen endoskopischen Instrument bevorzugt in einem Bereich von 5 bis 10 mm, wobei die Größe der Querschnittsfläche in erster Linie den Einsatzbedingungen, d. h. den Abmessungen des entsprechenden Körperkanals angepasst ist.

In einer weiteren Ausführungsform weist der Schaft des endoskopischen Instruments linienförmige Kontaktbereiche auf, die an der Schaftoberfläche gitternetzartig angeordnet sind. Beispielsweise sind die Flächen zwischen den Erhebungen nicht durchgehend konkav in Richtung der Schaftlängsachse eingerückt, sondern als eine Aneinanderreihung konkav gekrümmter Taschen, die von nicht eingerückten schmalen Stegen begrenzt werden, ausgebildet. Die auf diese Weise gebildete oberflächliche Gitterstruktur weist eine Vielzahl von Bereichen auf, die vollständig von linienförmigen Erhebungen eingeschlossen sind. Die Scheitellinien der Erhebungen bilden die Kontaktbereiche zu dem Körpergewebe, während die eingeschlossenen Bereiche von dem Körpergewebe beabstandet sind und so mit diesem nicht in Kontakt treten können. Wird der Schaft des endoskopischen Instruments vor dem Eingriff mit einem Gleitmittel beaufschlagt, bilden die tiefer liegenden Bereiche geschlossene Depots, aus denen das Gleitmittel nicht abfließen kann.

Es kann von Vorteil sein, die Kontaktbereiche in Richtung ihrer Längsausdehnung zu unterbrechen. So werden lange linienförmige Kontaktbereiche in eine Reihe voneinander beabstandeter Kontaktbereiche aufgeteilt, die sich in eine gemeinsame Ausdehnungsrichtung erstrecken. Hierdurch wird die Kontaktfläche zu dem umgebenden Körpergewebe weiter verkleinert.

Durch die oben beschriebenen Ausführungsformen ist es vorteilhaft möglich, die Schaftoberfläche so auszubilden, dass die Kontaktbereiche mit dem umgebenden Körper gegenüber einem zylindrischen Schaft mit gleicher radialer Querschnittsausdehnung um 55 bis 75 % reduziert sind. Entsprechend werden 55 bis 75% des umgebenden Körpergewebes von dem Endoskopschaft nicht kontaktiert. Da bei einem Bewegen des Schafts die Beanspruchung des umgebenden Gewebes durch Gleitreibung mit dem Schaft direkt proportional zur Größe der Kontaktfläche mit dem Schaft ist, verringert sich diese Beanspruchung anteilmäßig gleich. Dies hat zur Folge, dass das Gewebe beim Bewegen des endoskopischen Instruments deutlich weniger belastet wird.

Zu einem kleinsten Hülldurchmesser, d. h. dem Durchmesser eines durch die Scheitelpunkte der Vertiefungen verlaufenden gedachten Hüllkreises, steht die Höhe der Erhebungen, die die Kontaktbereiche mit dem Körpergewebe bilden, vorteilhaft in einem Verhältnis von 1:10 bis 1:20. Auf diese Weise werden die oben beschriebenen verbesserten Kontaktbedingungen geschaffen, ohne bei Instrumenten mit einem Hohlschaft den Innenraum des Schaftes bzw. dessen Innenquerschnitt deutlich einzuengen.

Die Ausbildung der linienförmig gegenüber dem übrigen Schaft erhabenen Bereiche kann entweder, wie bei dem eingangs beschriebenen Wellenprofil durch Verformung eines zylindrischen Hohlschaftes oder aber auch durch gezielten Materialauftrag auf einen zylindrischen Schaft erfolgen. Dabei kann der Materialauftrag durch Schweißen oder andere geeignete Auftragsverfahren erfolgen. In umgekehrter Weise können die Erhebungen aber auch durch abtragende Verfahren, wie beispielsweise Funkenerosion, erzeugt werden, indem der Bereich neben den erhabenen linienförmigen Bereichen abgetragen wird. Auch kann beispielsweise zur Erzeugung einer gitternetzartigen Struktur, ein zylindrischer Schaft mit einem solchen netzartigen Hüllschlauch überzogen werden.

Die Erfindung ist nachfolgend anhand von in der Zeichnung dargestellten Ausführungsbeispielen erläutert. Es zeigen
- Fig. 1.: in schematischer Querschnittsdarstellung einen in einem Körperkanal befindlichen Schaft eines endoskopischen Instruments mit einem polygonen Schaftquerschnitt,
- Fig. 2: in schematischer Querschnittsdarstellung einen in einem Körperkanal befindlichen Schaft eines erfindungsgemäßen endoskopischen Instruments mit einer wellenförmigen Außenquerschnittskontur.

Bei den in den Figuren dargestellten Schäften 2 u. 2' handelt es sich um Ausführungsformen eines (beliebigen) Hohlschafts eines hier nicht näher beschriebenen endoskopischen Instruments, der ein Hüllrohr 4 u. 4' aufweist, in dessen Innenraum 6 u. 6' die am distalen Schaftende austretenden Arbeitswerkzeuge (in der Zeichnung nicht dargestellt) geführt werden oder weitere Bauteile angeordnet sind.

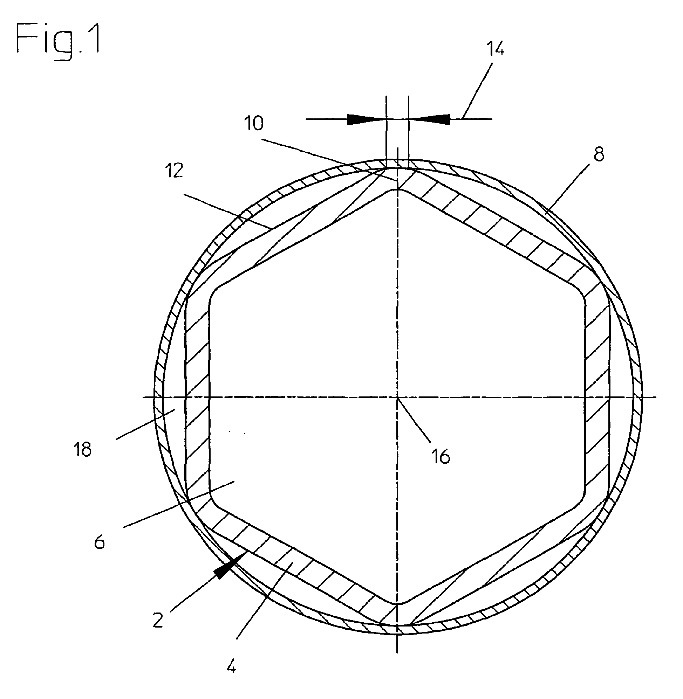

Der in Fig. 1 dargestellte Schaft 2, der nicht Teil der Erfindung ist, befindet sich in einem Körperkanal 8 und weist eine polygone Außenquerschnittskontur mit sechs Ecken 10 und dazwischenliegenden ebenen Flächensegmenten 12 auf. Die Anzahl der Ecken 8 beträgt in dem dargestellten Ausführungsbeispiel sechs, der Schaftquerschnitt kann jedoch je nach Anwendungsfall eine beliebige Anzahl von Ecken 10 aufweisen, wobei 5 bis 20-eckige Querschnittskonturen bevorzugt vorgesehen sind. Die Ecken 10 bilden Erhebungen mit Kontaktbereichen 14 zu dem umgebenden Körperkanal 8 aus, die sich an der Schaftoberfläche in Richtung dessen Längsachse 16 erstrecken. Auf diese Weise sind große Bereiche der Flächensegmente 12 von dem Körperkanal 8 abgerückt und bilden Zwischenräume 18 zwischen dem Körperkanal 8 und der Oberfläche des Schafts 2, so dass in diesem Bereich der Schaftoberfläche kein Kontakt mit dem Körpergewebe besteht. Um eine Traumatisierung des Körpergewebes des Körperkanals 8 zu verhindern, sind die Ecken 10 abgerundet ausgebildet.

In einer nicht dargestellten Ausführungsform des erfindungsgemäßen endoskopischen Instruments kann der Querschnitt eines Schafts eine 12- eckige Außenkontur aufweisen. Die zwischen den Ecken liegenden Flächensegmente sind konkav, d.h. in Richtung der Schaftlängsachse nach innen gekrümmt. Aufgrund dieser Krümmung bilden die Ecken Erhebungen aus, die gegenüber den in Fig. 1 dargestellten Erhebungen noch deutlicher hervorstehen. Die Schaftoberfläche weist so über ihren Umfang eine gleichmäßig verteilte Aufeinanderfolge von kammartigen Erhebungen und U-förmigen Senken auf, die in Richtung der Längsachse des Schafts verlaufen. Dabei berühren linienförmige Kontaktbereiche der Erhebungen die Wand des Körperkanals. Die zwischen den Erhebungen bzw. Ecken liegenden Flächensegmente sind wegen ihrer konkaven Krümmung noch weiter von der Wand des Körperkanals beabstandet. Entsprechend groß sind die von dem Körperkanal und den Flächensegmenten ausgebildeten Zwischenräume, die geeignete Depots für ein vor dem Anwenden des endoskopischen Instruments aufzutragendes Gleitmittel bilden.

Zweckmäßigerweise empfiehlt es sich bei diesem Ausführungsbeispiel, die Ecken abgerundet zu gestalten, da die Ecken aufgrund der Krümmung der diese Ecken bildenden Flächensegmente vergleichsweise spitz zulaufen und an der Oberfläche des Schafts dementsprechend scharfkantige Schneiden ausbilden können, durch die das den Körperkanal umgebende Körpergewebe traumatisiert werden könnte.

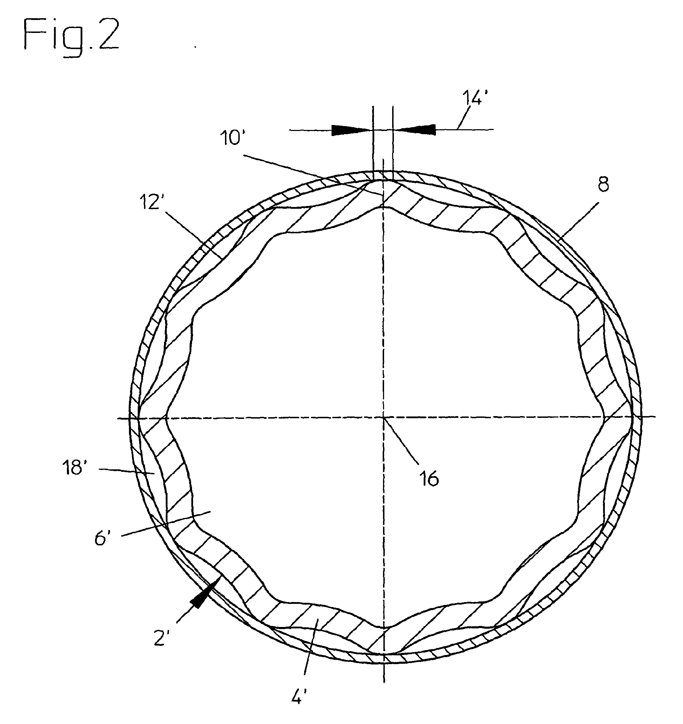

Eine besonders bevorzugte Ausführungsform des Schaftes sieht daher vor, die Ecken so abzurunden, dass die Eckenabrundung harmonisch in die konkave Krümmung der Flächensegmente übergeht, so dass die Außenquerschnittskontur des Schafts die in Fig. 2 dargestellte Wellenform aufweist.

In dieser Fig. 2 ist die Wandung des Hüllrohrs 4' im Querschnitt wellenförmig ausgebildet, so dass sinusförmig wechselnd Wellenberge und Wellentäler aufeinander folgen. Entsprechend ändert sich die radiale Querschnittsausdehnung des Schafts 2' periodisch und wächst kontinuierlich zu einem größten Wert an den Scheitelpunkten der Wellenberge, um sich anschließend kontinuierlich zu einem kleinsten Wert an den Scheitelpunkten der Wellentäler zu verringern. Der Abstand zwischen den Scheitelpunkten der Wellenberge und den Scheitelpunkten der Wellentäler ist in radialer Richtung in etwa so groß wie die Wanddicke des Hüllrohrs 4' und steht zur kleinsten radialen Querschnittsausdehnung des Hüllrohrs 4' bevorzugt in einem Verhältnis von 1:10 bis 1:20, so dass die zur Schaftlängsachse 16 gerichteten Wellentäler den Querschnitt des Innenraums 6' des Hüllrohrs 4' nicht so weit einengen, dass die Positionierung der in diesem Innenraum 6' geführten Arbeitswerkzeuge des endoskopischen Instruments behindert wird. Über den gesamten Umfang des Schafts 2' weisen in Umfangsrichtung benachbarte Scheitelpunkte bzw. Scheitelpunkte den gleichen Abstand voneinander auf.

Die Wellenberge und Wellentäler bilden an der Außenoberfläche des Schafts 2' jeweils 12 Erhebungen und Vertiefungen. Diese Erhebungen und Vertiefungen verlaufen linienförmig über die gesamte Länge des Schafts 2', und zwar parallel zur Schaftachse 16. Es ist aber auch möglich, nur Teilbereiche des Schafts 2' wellenförmig zu profilieren und beispielsweise den proximalen Endbereich des Schafts 2' zylindrisch oder prismenförmig auszubilden.

Die Scheitelbereiche der Erhebungen bilden die Kontaktflächen 14' mit dem umgebenden Körperkanal 8 aus, während die Vertiefungen und große Bereiche der Erhebungen mit dem Körpergewebe nicht in Berührung kommen und Zwischenräume 18' zwischen der Außenwandung des Schafts 2' und dem umgebenden Körperkanal 8 bilden, die als Depots für ein vor dem Einsatz des endoskopischen Instruments aufzutragendes Gleitmittel dienen können.

Obwohl in der Figur ein endoskopisches Instrument mit einem Hohlschaft dargestellt ist, kann auch die Schaftoberfläche von Instrumenten mit einem Vollschaft wie oben beschrieben gestaltet sein.

### Bezugszeichenliste

- 2, 2': Schaft
- 4,4': Hüllrohr
- 6,6': Innenraum
- 8: Körperkanal
- 10,10': Ecke
- 12, 12': Flächensegment
- 14, 14': Kontaktbereich
- 16: Schaftlängsachse
- 18, 18': Zwischenraum.

## Patentansprüche

1. Endoskopisches Instrument mit einem zum Einführen in eine Körperöffnung (8) vorgesehenen Schaft (2, 2'), wobei die Außenquerschnittskontur des Schafts (2, 2') Erhebungen aufweist, die auf den Eckpunkten eines Polygons liegen, so dass ein im Wesentlichen linienförmiger Kontakt (14, 14') zwischen Schaft (2, 2') und Körper (8) gebildet wird, **dadurch gekennzeichnet, dass** die Flächen (12, 12') zwischen den Erhebungen im Querschnitt zumindest teilweise konkav gekrümmt sind.

2. Endoskopisches Instrument nach Anspruch 1, **dadurch gekennzeichnet, dass** die Erhebungen des Schafts (2, 2') abgerundet sind.

3. Endoskopisches Instrument nach einem der vorangehenden Ansprüche, **dadurch gekennzeichnet, dass** der Schaft (2") an seinem Außenumfang ein Wellenprofil aufweist, wobei die Wellenberge (20) im Wesentlichen parallel zur Längsachse (16) des Schafts (2") verlaufen.

4. Endoskopisches Instrument nach einem der Ansprüche 1 oder 2, **dadurch gekennzeichnet, dass** die Außenquerschnittskontur des Schafts (2, 2') 5 bis 20 Erhebungen aufweist.

5. Endoskopisches Instrument nach einem der vorangehenden Ansprüche, **dadurch gekennzeichnet, dass** der Schaft linienförmige Kontaktbereiche (14, 14') aufweist, welche an der Schaftoberfläche gitternetzartig angeordnet sind.

6. Endoskopisches Instrument nach einem der vorangehenden Ansprüche, **dadurch gekennzeichnet, dass** die Kontaktbereiche (14, 14') in Richtung ihrer Längsausdehnung unterbrochen sind.

7. Endoskopisches Instrument nach einem der vorangehenden Ansprüche, **dadurch gekennzeichnet, dass** die größten radialen Querschnittsausdehnungen des Schafts einen Wert von 5 bis 10 mm aufweisen.

8. Endoskopisches Instrument nach einem der vorangehenden Ansprüche, **dadurch gekennzeichnet, dass** die Höhe der Erhebungen (20) bezogen auf den kleinsten Hülldurchmesser des Schafts (2") in einem Verhältnis von 1:10 bis 1:20 steht.

## Claims

1. An endoscopic instrument with a shank (2, 2') provided for introduction into a body opening (8), wherein the outer cross-sectional contour of the shank (2, 2') comprises raised parts which lie at the corner points of a polygon, so that an essentially linear contact (14, 14') between the shank (2, 2') and body (8) is formed, **characterised in that** the surfaces (12, 12') between the raised parts, in cross section, are at least partly curved in a concave manner.

2. An endoscopic instrument according to claim 1, **characterised in that** the raised parts of the shank (2, 2') are rounded off.

3. An endoscopic instrument according to one of the preceding claims, **characterised in that** the shank (2') on its outer periphery has a wave profile, wherein the wave hills (20) run essentially parallel to the longitudinal axis (16) of the shank (2').

4. An endoscopic instrument according to one of the claims 1 or 2, **characterised in that** the outer cross-sectional contour of the shank (2, 2') has 5 to 20 raised parts.

5. An endoscopic instrument according to one of the preceding claims, **characterised in that** the shank comprises linear contact regions (14, 14') which are arranged on the shank surface in the manner of a gridnet.

6. An endoscopic instrument according to one of the preceding claims, **characterised in that** the contact regions (14, 14') are interrupted in the direction of their longitudinal extension.

7. An endoscopic instrument according to one of the preceding claims, **characterised in that** the largest radial cross-sectional extensions of the shank have a value of 5 to 10 mm.

8. An endoscopic instrument according to one of the preceding claims, **characterised in that** the height of the raised parts with respect to the smallest enveloping diameter of the shank (2') is at a ratio of 1:10 to 1:20.

## Revendications

1. Instrument endoscopique comportant une tige (2, 2') prévue pour l'introduction dans une ouverture corporelle (8), le contour extérieur de la section transversale de la tige (2, 2') présentant des bossages qui se trouvent aux angles d'un polygone de telle sorte qu'il se forme un contact (14, 14') sensiblement linéaire entre la tige (2, 2') et le corps (8), **caractérisé en ce que** les surfaces (12, 12') entre les bossages ont en section transversale une courbure au moins partiellement concave.

2. Instrument endoscopique selon la revendication 1, **caractérisé en ce que** les bossages de la tige (2, 2') sont arrondis.

3. Instrument endoscopique selon l'une des revendications précédentes, **caractérisé en ce que** la tige (2") présente, sur son pourtour extérieur, un profil ondulé, les sommets (20) des ondulations étant sensiblement parallèles à l'axe longitudinal (16) de la tige (2").

4. Instrument endoscopique selon l'une des revendications 1 ou 2, **caractérisé en ce que** le contour transversal extérieur de la tige (2, 2') présente 5 à 20 bossages.

5. Instrument endoscopique selon l'une des revendications précédentes, **caractérisé en ce que** la tige présente des zones de contact (14, 14') linéaires qui sont disposées sur la surface de la tige sous la forme d'un réseau quadrillé.

6. Instrument endoscopique selon l'une des revendications précédentes, **caractérisé en ce que** les zones de contact (14, 14') sont interrompues dans le sens de leur extension longitudinale.

7. Instrument endoscopique selon l'une des revendications précédentes, **caractérisé en ce que** les plus grandes extensions transversales radiales de la tige ont une valeur de 5 à 10 mm.

8. Instrument endoscopique selon l'une des revendications précédentes, **caractérisé en ce que** la hauteur des bossages (20), rapportée au plus petit diamètre de la gaine de la tige (2"), se situe dans un rapport de 1:10 au 1:20.
